# EUROPÄISCHE PATENTSCHRIFT

(11) **EP 1 355 978 B1**
(45) Veröffentlichungstag und Bekanntmachung des Hinweises auf die Patenterteilung: **04.08.2004**
(21) Anmeldenummer: 01989554.9
(22) Anmeldetag: 03.12.2001
(51) Int. Cl.: C08J 7/12, C12N 11/06, C07K 17/06

(54) **YBERFLÄCHENFUNKTIONALISIERTES TRAGERMATERIAL, VERFAHREN FUR SEINE HERSTELLUNG SOWIE FESTPHASENSYNTHESEVERFAHREN**
SURFACE-FUNCTIONALISED CARRIER MATERIAL, METHOD FOR THE PRODUCTION THEREOF AND SOLID PHASE SYNTHESIS METHOD
MATERIAU SUPPORT A FONCTION DE SURFACE, SON PROCEDE DE PRODUCTION ET PROCEDE DE SYNTHESE EN PHASE SOLIDE

(30) Priorität: 22.12.2000 DE 10065788
(43) Veröffentlichungstag der Anmeldung: 29.10.2003
(73) Patentinhaber: Poly-An Gesellschaft zur Herstellung von Polymeren für spezielle Anwendungen und Analytik mbH, 13086 Berlin (DE)
(72) Erfinder: WESSIG, Pablo, 13353 Berlin (DE); BENDIG, Jürgen, 10369 Berlin (DE); SCHEDLER, Uwe, 10119 Berlin (DE)
(74) Vertreter: Schneider, Henry, Dipl.-Ing.
(86) Internationale Anmeldenummer: PCT/EP2001/014073
(87) Internationale Veröffentlichungsnummer: WO 2002/051917

(56) Entgegenhaltungen:
- WO-A-91/16425
- US-A- 4 340 482
- US-A- 5 240 747
- R.B. MERRIFIELD: "SOLID PHASE PEPTIDE SYNTHESIS" JOURNAL OF THE AMERICAN CHEMICAL SOCIETY, Bd. 85, 1963, Seiten 2149-2154, XP002197448 in der Anmeldung erwähnt

## Beschreibung

Die Erfindung betrifft ein oberflächenfunktionalisiertes Trägermaterial, bestehend aus einem Trägermaterial und mindestens einer, an einer polymeren Oberfläche von diesem kovalent gebundenen Linkerverbindung, ein Verfahren für seine Herstellung sowie ein Verfahren zur Festphasensynthese von Aminosäuren, Peptiden oder Molekülen mit mindestens einer Peptidstruktureinheit.

Es ist bekannt, die Synthese von Peptiden oder komplexeren Molekülen mit Peptidstruktureinheiten in Form so genannter Festphasensynthesen durchzuführen. Dafür wird eine Aminosäure, die quasi ein erstes molekulares Glied der herzustellenden Peptidsequenz darstellt, an ein nicht wasserlösliches Trägermaterial, dessen Oberfläche geeignete funktionelle Gruppen trägt, kovalent gebunden. Eine weitere Kettenverlängerung erfolgt, indem weitere Aminosäuren entsprechend der aufzubauenden Sequenz sukzessiv an die erste Aminosäure beziehungsweise an das freie Ende der entstehenden Peptidkette gebunden werden. Neben der reinen Kettenverlängerung ist es ferner bekannt, chemische Modifikationen an der immobilisierten Aminosäure beziehungsweise dem immobilisierten Peptid vorzunehmen. Als Basismaterial für die feste Phase (das Trägermaterial) wird überwiegend Polystyrol eingesetzt (siehe hierzu F. Z. Dörwald, Organic Synthesis on Solid Phases, Wiley-Verlag Chemie, Weinheim 2000, S. 414 ff).

Hinsichtlich einer Syntheserichtung des herzustellenden Peptides werden zwei Strategien unterschieden. In der Merrifield-Strategie, die auch als A-Typverlängerung bezeichnet wird, erfolgt eine Oberflächenfunktionalisierung des Polystyrols durch Derivatisierung mit Chlormethyl-, Hydroxymethyl- oder Acrylamid-Gruppen (R. B. Merrifield, J. Am. Chem. Soc. 1963, 85, S. 2149-2154; R. Arshady et al., J. Chem. Soc. Perkin Trans. I 1981, S. 529-537). Die kovalente Anknüpfung der ersten Aminosäure an diese Gruppen erfolgt über die Carboxylgruppe der Aminosäure, das heißt C-terminal. Der weitere Kettenaufbau schließt eine Kondensation der nächstfolgenden Aminosäure an die Aminogruppe (den N-Terminus) der bereits immobilisierten Aminosäure, beziehungsweise - bei weiterer Synthese - des immobilisierten Peptides ein. Gemäß der Merrifield-Strategie erfolgt demnach die Synthese vom C-zum N-Terminus des Peptides. Auch bei der sich aus dem Merrifield-Konzept abgeleiteten Boc-Strategie (R. Arshady et al., J. Chem. Soc. Perkin Trans. I 1981, 529-537) beziehungsweise der Fmoc-Strategie (L. A. Carpino, G. Y. Han, J. Org. Chem. 1972, 37, 3404-3409), bei denen die Aminogruppe der jeweils anzuknüpfenden Aminosäuren durch bestimmte Schutzgruppen geschützt wird, ist das Peptid letztendlich über die Carbonylfunktion der ersten Aminosäure an die feste Phase gebunden.

Hingegen werden bei der inversen Strategie (B-Typverlängerung) auf der Oberfläche von Polystyrol immobilisierte Chlorameisensäureester-Einheiten als Ausgangspunkt für die Peptidsynthese benutzt (R. L. Letsinger, M. J. Kornet, J. Am. Chem. Soc. 1963, 85, 2149-2154; R. Matsueda et al., J. Am. Chem. Soc. 1975, 97, 2573-2575). Die Synthese wird hier durch N-terminale Anknüpfung der als tert-Butylester geschützten Aminosäuren in der durch die Zielsequenz vorgegebenen Reihenfolge in Richtung des C-Terminus des Peptides durchgeführt.

Nachteilig an diesem Stand der Technik ist, dass abhängig von der gewünschten Synthesestrategie unterschiedlich funktionalisierte Trägermaterialien eingesetzt werden müssen, da die chemischen Eigenschaften der jeweiligen Oberflächenfunktion des Trägermaterials ausschließlich eine N-terminale oder eine C-terminale Anknüpfung der Aminosäure erlaubt.

Aufgabe der vorliegenden Erfindung ist es daher, ein oberflächenfunktionalisiertes Trägermaterial zur Verfügung zu stellen, das einem Anwender wahlweise eine C-terminale oder eine N-terminale Anbindung einer Aminosäure oder eines anderen Moleküls mit entsprechenden funktionellen Gruppen erlaubt. Es soll ferner ein Verfahren zur Herstellung des oberflächenfunktionalisierten Trägermaterials sowie ein Verfahren zur Festphasensynthese bereitgestellt werden.

Der erste Aspekt dieser Aufgabe wird durch ein oberflächenfunktionalisiertes Trägermaterial mit einer polymeren Oberfläche und mindestens einer an dieser kovalent gebundenen Linkerverbindung gemäß der allgemeinen Formel (I) gelöst 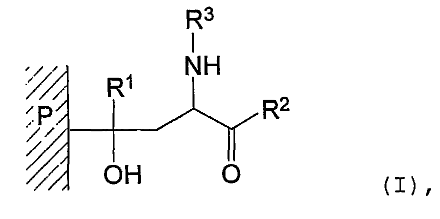 in der P die polymere Oberfläche bedeutet; R² die Bedeutung OR⁴ oder NR⁴R⁵ hat und R¹, R⁴ und R⁵ unabhängig voneinander H, eine Alkylgruppe oder eine Arylgruppe bedeuten; R³ H, eine Alkyl-, eine Aryl-, eine Acyl-, eine Alkoxycarbonyl- oder eine Aryloxycarbonylgruppe bedeutet; und die Alkyl-, Aryl-, Acyl-, Alkoxycarbonyl- und/oder Aryloxycarbonylgruppe der Reste R¹, R³, R⁴ und R⁵ unabhängig voneinander substituiert oder unsubstituiert sind. Die an das Trägermaterial kovalent gebundene Linkerverbindung lässt sich auf die Aminosäure Glycin zurückführen, deren Amino- und/oder Carboxylgruppe gegebenenfalls derivatisiert ist und die an ihrer C^{α}-Position über eine Hydroxyethyleinheit kovalent an die polymere Oberfläche des Trägermaterials gebunden ist. Dabei kann die Hydroxyethyleinheit an der, die Hydroxygruppe tragenden C-Position ebenfalls substituiert sein. Das erfindungsgemäße Material zeichnet sich demnach dadurch aus, dass es zwei freie Funktionen trägt, nämlich die Amino- und die Carboxylgruppe des Glycins, an denen ein Biomolekül, insbesondere eine Aminosäure, ankondensiert werden kann. Es handelt sich damit bei dem erfindungsgemäßen Material um ein zweifach oberflächenfunktionalisiertes Material.

Die Alkylgruppen der Reste R¹, R³, R⁴ und R⁵ sowie die Alkylreste der Acyl- oder der Alkoxycarbonylgruppe des Restes R³ sind vorzugsweise verzweigte oder unverzweigte, gesättigte oder ungesättigte C1- bis C20-Einheiten. Als Arylgruppen der Reste R¹, R³, R⁴ und R⁵ sowie der Arylrest der Aryloxycarbonylgruppe des Restes R³ können vorteilhaft Phenylgruppen eingesetzt werden.

Die polymere Oberfläche und/oder das Trägermaterial selbst ist gemäß einer vorteilhaften Ausgestaltung ein organisches Polymer, insbesondere Polypropylen, Polyethylen, Polysulfon, Polyethersulfon, Polystyrol, Polyvinylchlorid, Polyacrylnitril, Zellulose, Amylose, Agarose, Polyamid, Polyimid, Polytetrafluorethylen, Polyvinylidendifluorid, Polyester, Polycarbonat, Polyacrylat, Polyacrylamid oder ein Derivat von diesen oder ein Copolymer oder ein Blend von diesen. Daneben können jedoch auch anorganische und/oder mineralische Materialien als Trägermaterial eingesetzt werden, insbesondere Gläser, Silikate, keramische Materialien oder Metalle. Es ist ferner denkbar, Komposite aus einem oder mehreren anorganischen und/oder mineralischen Materialien und einem oder mehreren organischen Polymeren zu verwenden. Im Falle reiner anorganischer und/oder mineralischer Trägermaterialien kann eine Beschichtung mit einem der genannten organischen Polymermaterialien erforderlich sein, um eine Anbindung der Linkerverbindung zu ermöglichen.

Hinsichtlich seiner äußeren Gestalt kann das Trägermaterial in Form einer Membran, eines Films, einer Platte, einer Mikrotiterplatte, eines Reaktionsgefäßes, eines Objektträgers, einer Faser, einer Hohlfaser, eines Vlieses, eines Gewebes, eines Pulvers, eines Granulates oder in Form von Partikeln vorliegen. Dabei kann das Trägermaterial jeweils eine poröse oder nichtporöse Struktur aufweisen. Es ist besonders bevorzugt vorgesehen, dass das Trägermaterial in Form einer Membran mit einer symmetrischen oder asymmetrischen Porenstruktur vorliegt, wobei eine Porengröße im Bereich 1 nm bis 10 µm liegen kann.

Die Herstellung des oberflächenbifunktionalisierten Polymermaterials erfolgt vorzugsweise durch
(a) Aufbringen einer Linkerverbindung nach der allgemeinen Formel II 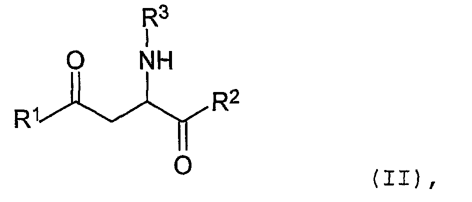 in der R¹, R² und R³ die obige Bedeutung haben, auf eine polymere Oberfläche (P) eines Trägermaterials, und
(b) Bestrahlung der Oberfläche mit Licht des UV-vis Spektralbereiches, wobei eine kovalente Bindung zwischen der Verbindung nach Formel (II) und der polymeren Oberfläche (P) unter Ausbildung des oberflächenfunktionalisierten Trägermaterials nach Formel (I) entsteht.

Das Aufbringen des Glycinderivates nach Formel (II) auf das Trägermaterial beziehungsweise seine polymere Oberfläche, welches aus den oben beschriebenen Materialien gewählt wird, erfolgt in Abhängigkeit von der äußeren Form des Trägermaterials durch Tränken, Befeuchten oder Beschichten.

Die Belichtung der Oberfläche kann - obwohl nicht zwingend erforderlich - besonders vorteilhaft in Gegenwart eines Sensibilisators durchgeführt werden. Auf diese Weise lässt sich die Ausbeute der Photoreaktion erhöhen. Die Bestrahlung erfolgt vorzugsweise mit Licht des Wellenlängenbereiches von 250 bis 500 nm. Dabei hängt die Wahl der verwendeten Wellenlänge oder des Wellenlängenbereiches in erster Linie von dem Rest R¹, der polymeren Oberfläche und dem Vorhandensein und der Art des Sensibilisators ab. Geeignete Lichtquellen sind beispielsweise Laser, UV-Röhren oder Quecksilberdampflampen, wobei gegebenenfalls der Wellenlängenbereich durch Verwendung geeigneter Filter begrenzt werden kann. Die lichtinduzierte Kopplung von Molekülen an polymeren Oberflächen ist beispielsweise aus der WO 91/16425 oder der EP 0 562 373 A2 bekannt und soll hier nicht näher erläutert werden.

Nach erfolgter photochemischer Umsetzung werden nicht umgesetzte Linkerverbindung, Nebenprodukte und gegebenenfalls der Sensibilisator durch Waschen mit einer Waschflüssigkeit wie Wasser, einem organischen Lösungsmittel oder einem Lösungsmittelgemisch entfernt und das funktionalisierte Trägermaterial getrocknet. Das getrocknete Produkt weist eine sehr gute Stabilität auf und kann bei Raumtemperatur über Wochen und Monate aufbewahrt werden.

Das erfindungsgemäße oberflächenfunktionalisierte Trägermaterial lässt sich zur kovalenten Immobilisierung von Biomolekülen, insbesondere von Aminosäuren, Peptiden oder Proteinen oder anderen Molekülen mit Amino- und/oder Carboxylgruppen verwenden.

Es kann ferner besonders vorteilhaft für die Festphasensynthese von Aminosäuren, Peptiden, Proteinen oder Molekülen mit mindestens einer Peptidstruktureinheit eingesetzt werden. Dabei kann eine erste, für die Synthese eingesetzte Aminosäure durch eine Peptidbindung wahlweise entweder zwischen der Aminogruppe der Aminosäure und der C1-Position der Linkerverbindung oder zwischen der Carboxylgruppe der Aminosäure und der Aminogruppe der Linkerverbindung an das Trägermaterial gebunden werden. Ist eine selektive C- oder N-terminale Anbindung erwünscht, kann die Verknüpfung in bekannter Weise durch Verwendung von chemischen Schutzgruppen zum Blockieren des N-Terminus oder des C-Terminus der Aminosäure und/oder der C1-Position beziehungsweise der Aminogruppe der Linkerverbindung gelenkt werden. Dabei wird der nicht anzubindende Terminus der Aminosäure beziehungsweise die nicht zu verbindende Funktion der Linkerverbindung durch die Schutzgruppe blockiert. Seitens der Linkerverbindung kann eine Steuerung der Anknüpfung des Biomoleküls durch geeignete Auswahl der Reste R² beziehungsweise R³ erfolgen.

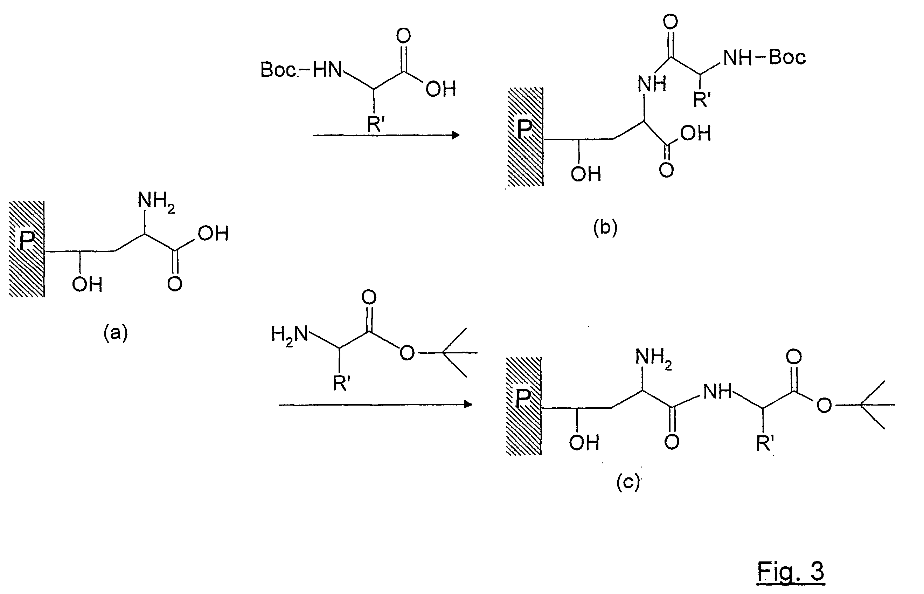

Die regiospezifische Immobilisierung einer Aminosäure ist in Figur 3 dargestellt, wobei die Reste R¹ und R³ der oberflächenimmobilisierten Linkerverbindung (a) jeweils Wasserstoff sind und der Rest R² eine Hydroxylgruppe ist. Gemäß dem oberen Zweig der Abbildung (b) erfolgt die Ankondensation der ersten Aminosäure, deren Aminofunktion mit einer tert-Butoxycarbonyl-Schutzgruppe (Boc) geschützt ist, mit ihrer freien Carboxylfunktion an die Aminogruppe des Trägermaterials unter Ausbildung einer Amidbindung. Dabei kann eine Aktivierung der Carboxylgruppe durch vorherige Umsetzung der Aminosäure mit Dicyclohexylcarbodiimid (DCCI) erzielt werden. Auch diese Maßnahme ist dem Fachmann bekannt. Eine Peptidkettenverlängerung der kovalent angeknüpften Aminosäure kann nun derart erfolgen, dass nach saurer hydrolytischer Entfernung der Boc-Schutzgruppe weitere N-terminal geschützte Aminosäuren an den nunmehr freien N-Terminus der ersten Aminosäure ankondensiert werden (A-Typverlängerung). Die einzelnen Verfahrensschritte Kupplung und Schutzgruppenabspaltung werden so oft wiederholt, bis die gewünschte Peptidsequenz vollständig aufgebaut vorliegt. Eine Abspaltung des fertigen Peptides erfolgt durch Zugabe von verdünnter Fluorwasserstoffsäure.

Die inverse Strategie (B-Typverlängerung) erfolgt gemäß dem unteren Zweig (c) der Figur 3. Hier ist die Carboxylgruppe der zu immobilisierenden Aminosäure durch einen tert-Butylester geschützt. Folglich kann eine Kondensation der Aminosäure mit dem oberflächenfunktionalisierten Trägermaterial nur über die Aminogruppe der Aminosäure an die Carboxylgruppe des Trägermaterials erfolgen. Für einen weiteren Peptidaufbau wird dann zunächst die tert-Butylgruppe durch Verseifung entfernt und weitere, entsprechend geschützte Aminosäuren ankondensiert.

Die Erfindung wird nachfolgend in Ausführungsbeispielen näher erläutert.

### Beispiel 1

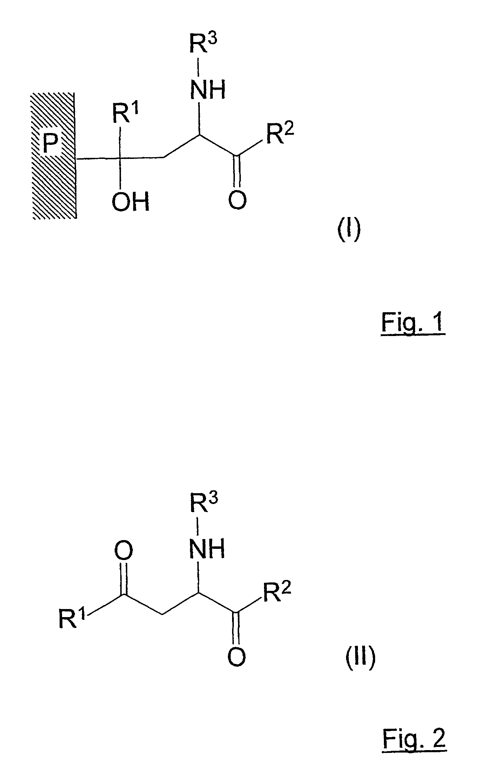

In eine 0,1 M Lösung von N-Boc-L-β-benzoylalanin, das heißt eine Aminosäure der allgemeinen Formel (II) (vergleiche Figur 2) mit R¹ = Phenyl, R² = OR⁴, R³ = t-Butoxycarbonyl (Boc) und R⁴ = H, in Dichlormethan wurde eine Polypropylen-Membran (Durchmesser 30 mm) 30 Minuten getaucht und anschließend im Hochvakuum bei 3·10⁻⁵ Torr für 30 Minuten getrocknet.

Die Membran wurde 30 Minuten mit dem Licht einer HBO(Höchstdruckbrenner) 500 im Abstand von 20 cm bei Einsatz eines Kantenfilters, der das Licht unterhalb 290 nm herausfiltert, bestrahlt.

Die Membran wurde anschließend fünfmal mit insgesamt 150 ml Dichlormethan gewaschen und 30 Minuten im Hochvakuum bei 3·10⁻⁵ Torr getrocknet.

Der Nachweis der Oberflächenfunktionalisierung entsprechend Formel (I) (vergleiche Figur 1), in der R¹ bis R⁴ die oben genannten Bedeutungen besitzen, erfolgte durch Vergleich der FT-IR-Spektren für die Polypropylen-Membran vor und nach der Behandlung.

### Beispiel 2

In eine 0,1 M Lösung von N-Boc-L-β-benzoylalaninmethylester, das heißt eine Aminosäure der allgemeinen Formel (II) mit R¹ = Phenyl, R² = OR⁴, R³ = t-Butoxycarbonyl (Boc) und R⁴ = Methyl, in Dichlormethan wurde eine Polypropylen-Membran (Durchmesser 30 mm) 30 Minuten getaucht und anschließend im Hochvakuum bei 3·10⁻⁵ Torr für 30 Minuten getrocknet.

Die Membran wurde 30 Minuten mit dem Licht einer HBO 500 im Abstand von 20 cm bei Einsatz eines Kantenfilters, der das Licht unterhalb 290 nm herausfiltert, bestrahlt.

Die Membran wurde anschließend fünfmal mit insgesamt 150 ml Dichlormethan gewaschen und 30 Minuten im Hochvakuum bei 3·10⁻⁵ Torr getrocknet.

Der Nachweis der Oberflächenfunktionalisierung entsprechend Formel (I), in der R¹ bis R⁴ die oben genannten Bedeutungen besitzen, erfolgte durch Vergleich der FT-IR-Spektren für die Polypropylen-Membran vor und nach der Behandlung.

### Beispiel 3

Eine Polypropylen-Membran (Durchmesser 30 mm) wurde in einer Glasschale, gefüllt mit einer 0,1 M Lösung von N-Boc-L-β-benzoylalanin, das heißt eine Aminosäure der allgemeinen Formel (II) mit R¹ = Phenyl, R² = OR⁴, R³ = t-Butoxycarbonyl (Boc) und R⁴ = H, in Benzol 60 Minuten mit dem Licht einer HBO 500 im Abstand von 20 cm bei Einsatz eines Umlenkspiegels und eines Kantenfilters, der das Licht unterhalb 290 nm herausfiltert, bestrahlt.

Die Membran wurde anschließend einmal mit 50 ml Benzol und zweimal mit 50 ml Dichlormethan gewaschen und 30 Minuten im Hochvakuum bei 3.10⁻⁵ Torr getrocknet.

Der Nachweis der Oberflächenfunktionalisierung entsprechend Formel (I), in der R¹ bis R⁴ die oben genannten Bedeutungen besitzen, erfolgte durch Vergleich der FT-IR-Spektren für die Polypropylen-Membran vor und nach der Behandlung.

## Patentansprüche

1. Oberflächenfunktionalisiertes Trägermaterial mit einer polymeren Oberfläche und mindestens einer an dieser kovalent gebundenen Linkerverbindung gemäß der allgemeinen Formel (I) 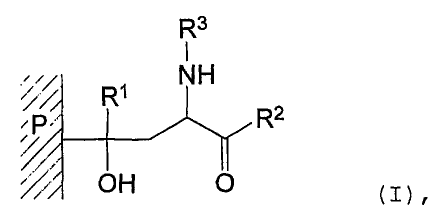 in der P die polymere Oberfläche bedeutet; R² die Bedeutung OR⁴ oder NR⁴R⁵ hat und R¹, R⁴ und R⁵ unabhängig voneinander H, eine Alkylgruppe oder eine Arylgruppe bedeuten; R³ H, eine Alkyl-, eine Aryl-, eine Acyl-, eine Alkoxycarbonyl- oder eine Aryloxycarbonylgruppe bedeutet; und die Alkyl-, Aryl-, Acyl-, Alkoxycarbonyl- und/oder Aryloxycarbonylgruppe der Reste R¹, R³, R⁴ und R⁵ unabhängig voneinander substituiert oder unsubstituiert sind.

2. Oberflächenfunktionalisiertes Trägermaterial nach Anspruch 1, **dadurch gekennzeichnet, dass** die polymere Oberfläche (P) und/oder das Trägermaterial ein organisches Polymer ist.

3. Oberflächenfunktionalisiertes Trägermaterial nach Anspruch 2, **dadurch gekennzeichnet, dass** das organische Polymer Polypropylen, Polyethylen, Polysulfon, Polyethersulfon, Polystyrol, Polyvinylchlorid, Polyacrylnitril, Zellulose, Amylose, Agarose, Polyamid, Polyimid, Polytetrafluorethylen, Polyvinylidendifluorid, Polyester, Polycarbonat, Polyacrylat, Polyacrylamid oder ein Derivat von diesen ist oder ein Copolymer oder ein Blend von diesen.

4. Oberflächenfunktionalisiertes Trägermaterial nach Anspruch 1, **dadurch gekennzeichnet, dass** das Trägermaterial ein anorganisches und/oder mineralisches Material ist.

5. Oberflächenfunktionalisiertes Trägermaterial nach Anspruch 4, **dadurch gekennzeichnet, dass** das Trägermaterial ein Glas, ein Silikat, ein keramisches Material oder ein Metall ist.

6. Oberflächenfunktionalisiertes Trägermaterial nach einem der Ansprüche 2 bis 5, **dadurch gekennzeichnet, dass** das Trägermaterial ein Komposit aus mindestens einem anorganischen und/oder mineralischen Material und mindestens einem organischen Polymer ist.

7. Oberflächenfunktionalisiertes Trägermaterial nach einem der vorhergehenden Ansprüche, **dadurch gekennzeichnet, dass** das Trägermaterial in Form einer Membran, eines Films, einer Platte, einer Mikrotiterplatte, eines Reaktionsgefäßes, eines Objektträgers, einer Faser, einer Hohlfaser, eines Vlieses, eines Gewebes, eines Pulvers, eines Granulates, oder von Partikeln vorliegt und jeweils porös oder nichtporös sein kann.

8. Oberflächenfunktionalisiertes Trägermaterial nach Anspruch 7, **dadurch gekennzeichnet, dass** das Trägermaterial in Form einer Membran mit einer symmetrischen oder asymmetrischen Porenstruktur vorliegt.

9. Oberflächenfunktionalisiertes Trägermaterial nach Anspruch 7 oder 8, **dadurch gekennzeichnet, dass** eine Porengröße 1 nm bis 10 µm beträgt.

10. Oberflächenfunktionalisiertes Trägermaterial nach einem der vorhergehenden Ansprüche, **dadurch gekennzeichnet, dass** die Alkylgruppen der Reste R¹, R³, R⁴ und R⁵ und der Acyl- und der Alkoxycarbonylgruppe des Restes R³ C1- bis C20-Alkyleinheiten sind.

11. Oberflächenfunktionalisiertes Trägermaterial nach einem der vorhergehenden Ansprüche, **dadurch gekennzeichnet, dass** die Arylgruppen der Reste R¹, R³, R⁴ und R⁵ und der Aryloxycarbonylgruppe des Restes R³ eine Phenylgruppe ist.

12. Verfahren zur Herstellung eines oberflächenfunktionalisierten Trägermaterials gemäß Formel (I) 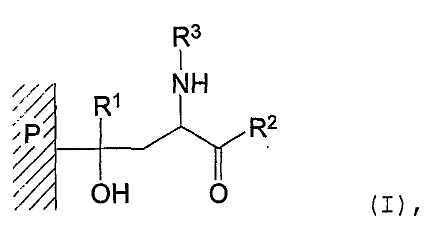 wobei P die polymere Oberfläche des Trägermaterials bedeutet; R² die Bedeutung OR⁴ oder NR⁴R⁵ hat und R¹, R⁴ und R⁵ unabhängig voneinander H, eine Alkylgruppe oder eine Arylgruppe bedeuten; R³ H, eine Alkyl-, eine Aryl-, eine Acyl-, eine Alkoxycarbonyl- oder eine Aryloxycarbonylgruppe bedeutet; und die Alkyl-, Aryl-, Acyl-, Alkoxycarbonyl- und/oder Aryloxycarbonylgruppe der Reste R¹, R³, R⁴ und R⁵ unabhängig voneinander substituiert oder unsubstituiert sind, mit den Schritten
(a) Aufbringen einer Linkerverbindung nach der allgemeinen Formel (II) 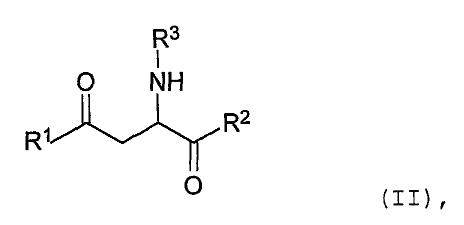 in der R¹, R² und R³ die obige Bedeutung haben, auf eine polymere Oberfläche (P) eines Trägermaterials ,
(b) Bestrahlung der Oberfläche mit Licht des UV-vis Spektralbereiches, wobei eine kovalente Bindung zwischen der Linkerverbindung nach Formel (II) und der polymeren Oberfläche (P) unter Ausbildung des oberflächenfunktionalisierten Trägermaterials nach Formel (I) entsteht.

13. Verfahren nach Anspruch 12, **dadurch gekennzeichnet, dass** die Bestrahlung mit Licht eines Wellenlängenbereiches von 250 bis 500 nm erfolgt.

14. Verfahren nach Anspruch 13, **dadurch gekennzeichnet, dass** als Lichtquelle Laser, UV-Röhren oder Quecksilberdampflampen gegebenenfalls unter Verwendung geeigneter Filter eingesetzt werden.

15. Verfahren nach einem der Ansprüche 12 bis 14, **dadurch gekennzeichnet, dass** die Bestrahlung der Oberfläche in Gegenwart oder in Abwesenheit eines Sensibilisators erfolgt.

16. Verfahren nach einem der Ansprüche 12 bis 15, **dadurch gekennzeichnet, dass** nach der Bestrahlung nicht umgesetzte Substanzen durch Waschen mit einer Waschflüssigkeit entfernt werden.

17. Verfahren nach Anspruch 16, **dadurch gekennzeichnet, dass** als Waschflüssigkeit Wasser, ein organisches Lösungsmittel oder ein Lösungsmittelgemisch verwendet wird.

18. Verfahren nach einem der Ansprüche 12 bis 17, **dadurch gekennzeichnet, dass** als polymere Oberfläche (P) und/oder Trägermaterial ein Material nach einem der Ansprüche 2 bis 9 verwendet wird.

19. Verwendung eines oberflächenfunktionalisierten Trägermaterials gemäß der allgemeinen Formel (I) nach einem der Ansprüche 1 bis 11 zur kovalenten Immobilisierung von Biomolekülen, insbesondere von Aminosäuren, Peptiden oder Proteinen oder Molekülen mit Amino- und oder Carboxylgruppen.

20. Verfahren zur Synthese von Aminosäuren, Peptiden, Proteinen oder Molekülen mit mindestens einer Peptidstruktureinheit an Festphasen, wobei eine erste, für die Synthese einzusetzende Aminosäure kovalent an ein oberflächenfunktionalisiertes Trägermaterial gebunden wird und eine Kettenverlängerung durch sukzessives Anknüpfen weiterer Aminosäuren und/oder eine chemische Modifizierung erfolgt, **dadurch gekennzeichnet, dass** ein oberflächenfunktionalisiertes Trägermaterial gemäß der allgemeinen Formel (I) nach einem der Ansprüche 1 bis 11 als Festphase verwendet wird.

21. Verfahren nach Anspruch 20, **dadurch gekennzeichnet, dass** die erste für die Synthese eingesetzte Aminosäure durch eine Peptidbindung wahlweise entweder zwischen der Aminogruppe der Aminosäure und der C1-Position der Linkerverbindung oder zwischen der Carboxylgruppe der Aminosäure und der Aminogruppe der Linkerverbindung an das Trägermaterial gebunden wird.

22. Verfahren nach Anspruch 21, **dadurch gekennzeichnet, dass** eine Steuerung der N- oder C-terminalen Anbindung der Aminosäure an die Linkerverbindung durch Blockieren der Aminogruppe oder der Carboxylgruppe der Aminosäure und/oder der C1-Position oder der Aminogruppe der Linkerverbindung mit chemischen Schutzgruppen erfolgt.

## Claims

1. Surface-functionalised carrier material with a polymer surface and, bound to it via a covalent bond, at least one linker compound, according to the general formula (I)
[please refer to source text for general formula]
wherein P denotes the polymer surface; R² denotes OR⁴ or NR⁴R⁵ and R¹, R⁴ and R⁵ independently of one another denote H, an alkyl group or an aryl group; R³ denotes H, an alkyl, aryl, acyl, alkoxycarbonyl or aryloxycarbonyl group; and the alkyl, aryl, acyl, alkoxycarbonyl and/or aryloxycarbonyl group on the residues R¹, R³, R⁴ and R⁵ are substituted or unsubstituted independently of one another.

2. Surface-functionalised carrier material according to claim 1, **characterised in that** the polymer surface (P) and/or the carrier material is an organic polymer.

3. Surface-functionalised carrier material according to claim 2, **characterised in that** the organic polymer is polypropylene, polyethylene, polysulfone, polyethersulfone, polystyrene, polyvinyl chloride, polyacrylnitrile, cellulose, amylose, agarose, polyamide, polyimide, polytetrafluoroethylene, polyvinylidene difluoride, polyester, polycarbonate, polyacrylate, polyacrylamide or a derivative of the latter, or a copolymer or a blend of the latter.

4. Surface-functionalised carrier material according to claim 1, **characterised in that** the carrier material is an inorganic and/or mineral material.

5. Surface-functionalised carrier material according to claim 4, **characterised in that** the carrier material is a glass, a silicate, a ceramic material or a metal.

6. Surface-functionalised carrier material according to any one of claims 2 to 5, **characterised in that** the carrier material is a composite made from at least one inorganic and/or mineral material and at least one organic polymer.

7. Surface-functionalised carrier material according to any one of the preceding claims, **characterised in that** the carrier material is present in the form of a membrane, a film, a plate, a micro-titre plate, a reaction vessel, a slide, a fibre, a hollow fibre, a fleece, a fabric, a powder, a granulate or particles each of which may be porous or non-porous.

8. Surface-functionalised carrier material according to claim 7, **characterised in that** the carrier material is present in the form of a membrane with a symmetrical or asymmetrical pore structure.

9. Surface-functionalised carrier material according to claim 7 or 8, **characterised in that** a pore size is 1 nm to 10 µm.

10. Surface-functionalised carrier material according to any one of the preceding claims, **characterised in that** the alkyl groups of the residues R¹, R³, R⁴ and R⁵ and the acyl and alkoxycarbonyl group of the residue R³ are C1 to C20 alkyl units.

11. Surface-functionalised carrier material according to any one of the preceding claims, **characterised in that** the aryl group of the residues R¹, R³, R⁴ and R⁵ and the aryloxycarbonyl group of the residue R³ is a phenyl group.

12. Method for the manufacture of a surface-functionalised carrier material according to formula (I)
[please refer to source text for general formula]
wherein P denotes the polymer surface of the carrier material; R² denotes OR⁴ or NR⁴R⁵ and, independently of one another, R¹, R⁴ and R⁵ denote H, an alkyl group or an aryl group; R³ denotes H, an alkyl, aryl, acyl, alkoxycarbonyl or aryloxycarbonyl group; and the alkyl, aryl, acyl, alkoxycarbonyl and/or aryloxycarbonyl group of the residues R¹, R³, R⁴ and R⁵ are substituted or unsubstituted independently of one another, comprising the following steps:
(a) application of a linker compound according to the general formula (II)
[please refer to source text for general formula]
wherein R¹, R² and R³ have the meanings indicated above, onto a polymer surface (P) of a carrier material,
(b) irradiation of the surface with light of the UV-vis spectral range, wherein a covalent bond is formed between the linker compound according to formula (II) and the polymer surface (P) leading to the formation of the surface-functionalised carrier material according to formula (I).

13. Method according to claim 12, **characterised in that** the irradiation is carried out with light of a wavelength range from 250 to 500 nm.

14. Method according to claim 13, **characterised in that** laser, UV-tubes or mercury-vapour lamps are used as the light source, optionally with appropriate filters.

15. Method according to any one of claims 12 to 14, **characterised in that** the irradiation of the surface is carried out in the presence or in the absence of a sensitiser.

16. Method according to any one of claims 12 to 15, **characterised in that** after the irradiation, any unconverted substances are removed by washing with a washing fluid.

17. Method according to claim 16, **characterised in that** water, an organic solvent or a solvent mixture are used as the washing fluid.

18. Method according to any one of claims 12 to 17, **characterised in that** a material according to any one of claims 2 to 9 is used as the polymer surface (P) and/or carrier material.

19. Use of a surface-functionalised carrier material of the general formula (I) according to any one of claims 1 to 11, for the covalent immobilisation of biomolecules, especially amino acids, peptides or proteins or molecules with amino or carboxyl groups.

20. Method for the synthesis of amino acids, peptides, proteins or molecules with at least one peptide unit on solid phases, wherein a first amino acid to be used for the synthesis is bound via a covalent bond to a surface-functionalised carrier material and a chain extension takes place through successive connection of further amino acids and/or through chemical modification, **characterised in that** a surface-functionalised carrier material according to the general formula (I) as claimed in claims 1 to 11 is used as a solid phase.

21. Method according to claim 20, **characterised in that** the first amino acid used for the synthesis is bound to the carrier material via a peptide bond optionally either between the amino group of the amino acid and the C1-position of the linker compound or between the carboxyl group of the amino acid and the amino group of the linker compound.

22. Method according to claim 21, **characterised in that** a control of the N-terminal or C-terminal bonding of the amino acid to the linker compound takes place by blocking the amino group or the carboxyl group of the amino acid and/or the C1-position or the amino group of the linker compound with chemical protecting groups.

## Revendications

1. Matériau-support rendu fonctionnel à sa surface, comprenant une surface polymère et au moins un composé linker lié par covalence à cette surface conformément à la formule générale (I) 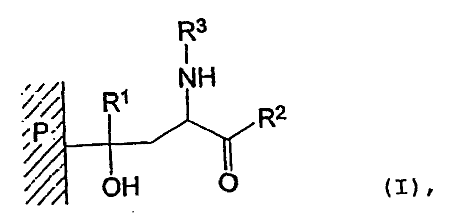 dans laquelle P désigne la surface polymère ; R² a pour définition OR⁴ ou NR⁴R⁵ et R¹, R⁴ et R⁵ représentent, indépendamment les uns des autres, H, un groupe alkyle ou un groupe aryle ; R³ représente H, un groupe alkyle, un groupe aryle, un groupe acyle, un groupe alkoxycarbonyle ou un groupe aryloxycarbonyle ; et les groupes alkyle, aryle, acyle, alkoxycarbonyle et/ou aryloxycarbonyle des restes R¹, R³, R⁴ et R⁵ sont, indépendamment les uns des autres, substitués ou non substitués.

2. Matériau-support rendu fonctionnel à sa surface suivant la revendication 1, **caractérisé en ce que** la surface polymère (P) et/ou le matériau-support sont un polymère organique.

3. Matériau-support rendu fonctionnel à sa surface suivant la revendication 2, **caractérisé en ce que** le polymère organique est un polypropylène, un polyéthylène, une polysulfone, une polyéthersulfone, un polystyrène, un polymère de chlorure de vinyle, un polymère d'acrylonitrile, la cellulose, l'amylose, l'agarose, un polyamide, un polyimide, un polymère de tétrafluoréthylène, un polymère de diflucrure de vinylidène, un polyester, un polycarbonate, un polyacrylate, un polyacrylamide ou un dérivé de ces composés ou un copolymère ou un mélange de ces composés.

4. Matériau-support rendu fonctionnel à sa surface suivant la revendication 1, **caractérisé en ce qu'**il est un matériau inorganique et/ou minéral.

5. Matériau-support rendu fonctionnel à sa surface suivant la revendication 4, **caractérisé en ce qu'**il est un verre, un silicate, un matériau céramique ou un métal.

6. Matériau-support rendu fonctionnel à sa surface suivant l'une des revendications 2 à 5, **caractérisé en ce qu'**il est un composite d'au moins une matière inorganique et/ou minérale et d'au moins un polymère organique.

7. Matériau-support rendu fonctionnel à sa surface suivant l'une des revendications précédentes, **caractérisé en ce qu'**il se présente sous forme d'une membrane, d'un film, d'une plaque, d'une plaque de microtitrage, d'un récipient de réaction, d'un porte-objet, d'une fibre, d'une fibre creuse, d'un voile, d'un tissu, d'une poudre, d'un produit granulé ou de particules et peut dans chaque cas être poreux ou non poreux.

8. Matériau-support rendu fonctionnel à sa surface suivant la revendication 7, **caractérisé en qu**'il se présente sous forme d'une membrane à structure poreuse symétrique ou asymétrique.

9. Matériau-support rendu fonctionnel à sa surface suivant la revendication 7 ou 8, **caractérisé par** un diamètre des pores allant de 1 nm à 10 µm.

10. Matériau-support rendu fonctionnel à sa surface suivant l'une des revendications précédentes, **caractérisé en ce que** les groupes alkyle des restes R¹, R³, R⁴ et R⁵ et des groupes acyle et alkoxycarbonyle du reste R³ sont des motifs alkyle en C₁ à C₂₀.

11. Matériau-support rendu fonctionnel à sa surface suivant l'une des revendications précédentes, **caractérisé en ce que** les groupes aryle des restes R¹, R³, R⁴ et R⁵ et du groupe aryloxycarbonyle du reste R³ sont représentés par un groupe phényle.

12. Procédé de production d'un matériau-support rendu fonctionnel à sa surface conformément à la formule (I) 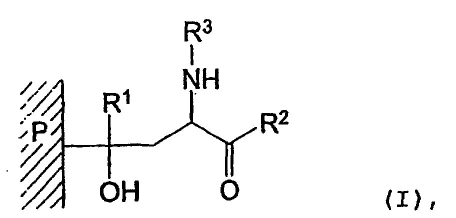 dans laquelle P désigne la surface polymère du matériau-support ; R² a pour définition OR⁴ ou NR⁴R⁵ et R¹, R⁴ et R⁵ représentent, indépendamment les uns des autres, H, un groupe alkyle ou un groupe aryle ; R³ représente H, un groupe alkyle, un groupe aryle, un groupe acyle, un groupe alkoxycarbonyle ou un groupe aryloxycarbonyle ; et les groupes alkyle, aryle, acyle, alkoxycarbonyle et/ou aryloxycarbonyle des restes R¹, R³, R⁴ et R⁵ sont, indépendamment les uns des autres, substitués ou non substitués, comprenant les étapes suivantes :
(a) application d'un composé linker de formule générale (II) 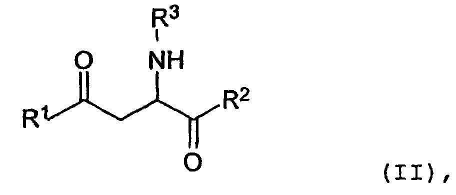 dans laquelle R¹, R² et R³ ont la définition indiquée ci-dessus, sur une surface polymère (P) d'un matériau-support,
(b) irradiation de la surface avec de la lumière de la plage spectrale allant de l'ultraviolet à la lumière visible, pour produire une liaison de covalence entre le composé linker de formule (II) et la surface polymère (P) avec réalisation du matériau-support fonctionnalisé à sa surface répondant à la formule (I).

13. Procédé suivant la revendication 12, **caractérisé en ce que** l'irradiation est effectuée avec de la lumière d'une longueur d'onde comprise dans la plage de 250 à 500 nm.

14. Procédé suivant la revendication 13, **caractérisé en ce qu'**on utilise comme source lumineuse un laser, des tubes UV ou des lampes à vapeur de mercure, le cas échéant avec utilisation de filtres convenables.

15. Procédé suivant l'une des revendications 12 à 14, **caractérisé en ce que** l'irradiation de la surface est effectuée en présence ou en l'absence d'un sensibilisateur.

16. Procédé suivant l'une des revendications 12 à 15, **caractérisé en ce que** les substances n'ayant pas réagi sont éliminées après l'irradiation par lavage avec un liquide.

17. Procédé suivant la revendication 16, **caractérisé en ce qu'**on utilise comme liquide de lavage l'eau, un solvant organique ou un mélange de solvants.

18. Procédé suivant l'une des revendications 12 à 17, **caractérisé en ce qu'**on utilise comme surface polymère (P) et/ou comme matériau-support un matériau selon l'une des revendications 2 à 9.

19. Utilisation d'un matériau-support rendu fonctionnel à sa surface de formule générale (I) suivant l'une des revendications 1 à 11 pour l'immobilisation par covalence de biomolécules, en particulier d'aminoacides, de peptides ou de protéines ou de molécules portant des groupes amino et/ou carboxyle.

20. Procédé de synthèse d'aminoacides, de peptides, de protéines ou de molécules portant au moins un motif structural peptidique sur des phases solides, dans lequel un premier aminoacide à utiliser pour la synthèse est lié par covalence à un matériau-support rendu fonctionnel à sa surface et un allongement de chaîne est effectué par liaison successive d'autres aminoacides et/ou par une modification chimique, **caractérisé en ce qu'**un matériau-support rendu fonctionnel à sa surface conformément à la formule générale (I) selon l'une des revendications 1 à 11, est utilisé comme phase solide.

21. Procédé suivant la revendication 20, **caractérisé en ce que** le premier aminoacide utilisé pour la synthèse est lié au matériau-support par une liaison peptidique au choix entre le groupe amino de l'aminoacide et la position C1 du composé linker ou entre le groupe carboxyle de l'aminoacide et le groupe amino du composé linker.

22. Procédé suivant la revendication 21, **caractérisé en ce qu'**une orientation de la liaison de l'extrémité terminale N ou C de l'aminoacide au composé linker est effectuée par blocage du groupe amino ou du groupe carboxyle de l'aminoacide et/ou de la position C1 ou du groupe amino du composé linker par des groupes chimiques protecteurs.
